# EUROPEAN PATENT APPLICATION

(11) **EP 3 228 451 A1**
(43) Date of publication of application: **11.10.2017**
(21) Application number: 15866235.3
(22) Date of filing: 23.09.2015
(51) Int. Cl.: B32B 7/12, A61F 13/42, B32B 27/00, B29C 47/06, B32B 5/00, B32B 37/00

(54) **METHOD FOR PRODUCING A LAMINAR PRODUCT PARTICULARLY SUITABLE FOR MANUFACTURING EXTERNAL COVERS OF SANITARY TOWELS AND NAPPIES, AND PRODUCT PRODUCED USING THIS METHOD**

(30) Priority: 05.12.2014 ES 201431801
(71) Applicant: KLONER, SL, 08392 San Andreu de Llavaneres (ES)
(72) Inventor: CAMPOS BECEIRO, Alberto, E-08392 Sant Andreu de Llavaneres (Barcelona) (ES)
(74) Representative: Oficina Ponti, SLP
(86) International application number: PCT/ES2015/070694
(87) International publication number: WO 2016/087692

(57) **Abstract**

The invention relates to a method for producing a laminar product suitable for manufacturing external covers of sanitary towels and nappies, formed by a polymeric coating deposited over the surface of a non-woven fabric substrate, as well as to the laminar product obtained using said method. The method comprises the following steps: obtaining adhesive polymeric mixtures, feeding the mixtures, in a melted state, to a curtain-type applicator, coating the substrate with the adhesive polymeric mixtures, and winding the laminar product obtained. The product obtained comprises a polymeric coating bonded to the non-woven fabric substrate, wherein the polymeric coating consists of an adhesive polymeric layer that has a reduced thickness, ranging between 1 and 10 micra, said product being suitable for medical and personal hygiene applications.

## Description

### Object of the invention.

As expressed in the title of this specification, the present invention relates to a method for producing a laminar product of the type formed by a polymeric coating deposited over the surface of a non-woven fabric substrate, said product being particularly suitable for manufacturing external covers of sanitary towels and nappies.

Another objective of the present invention is the laminar product produced using said method, which comprises a polymeric coating bonded, on one of its sides, to a non-woven fabric substrate, where said polymeric coating consists of an adhesive polymeric layer with a reduced thickness, suitable breathability values (water vapour permeability) and a barrier designed to prevent the loss of fluids. This adhesive polymeric layer lacks a porous structure and, consequently, the barrier against the loss of fluids is independent from the pore size and the ambient temperature, making said laminar product suitable to be used in the manufacturing of external covers of sanitary towels and nappies.

### Field of application of the invention.

The field of application of the invention is framed within the technical sector of polymeric-type coatings used in the manufacturing of laminar products designed to be used in medical and personal hygiene applications.

### Background of the invention.

There are several types of products used as covers of sanitary towels and nappies in the market:
a) Mono- or multi-layer polyethylene films.
   These layers may be breathable or non-breathable, and their breathability is obtained by forming micro-pores by means of post-stretching of the extruded polymeric film highly loaded with calcium carbonate particles. The thicknesses of this type of films range between 18 and 30 micra (16 to 32 g/m²). The external sides of these films are usually printed with several colours.
b) Film laminates over a non-woven fabric substrate, manufactured according to the preceding section. These covers may also be breathable or non-breathable, depending on the film, which is subsequently laminated over the substrate by means of a hot-melt adhesive, usually applied by means of the habitual techniques for applying hot-melt adhesives over flat substrates, such as spraying, with or without compressed air, extrusion of adhesive fibres with various deposition geometries, or application by means of linear nozzles. This lamination operation is performed in a separate process from the manufacturing of the film.
   The most habitual substrate is a non-woven fabric obtained by means of a spun-melt process, with a surface weight ranging between 10 and 15 g/m².
   The laminate usually has a surface weight ranging between 22 g/m² and 26 g/m² for non-breathable products, and between 28 g/m² and 36 g/m² for breathable products, including the adhesive layer, whose surface weight ranges between 2 and 4 g/m².
   These laminates are usually printed on the side that is in contact with the non-woven fabric substrate.
c) Direct coatings by means of extrusion over a non-woven fabric substrate such as the one described in the preceding section, using polymeric resins, generally polyethylene resins. They may also be breathable, by applying the necessary stretching to the coated woven product to generate pores in the polymeric layer.

In general, the surface weight of the substrate is not lower than 14 g/m², because, at lower surface weights, an excess opening of the structure, caused by the temperature conditions and the stresses on the product during the process, leads to a break in the coating continuity and the transfer of melted polymer towards the opposite surface.

On the other hand, the surface weight of the polymeric coating must be sufficient to fill the substrate roughness and prevent a break in the coating continuity. With the habitual substrates obtained by means of spun-melt-type processes, a continuous coating requires a minimum surface weight of 10-12 g/m².

Consequently, the total surface weight of this type of product usually ranges between 24 and 28 g/m².

Moreover, it is worth noting that this coating method is not suitable for printing on the side that is in contact with the non-woven fabric substrate, nor on the internal side of the product, as is habitually done in the market, since the opacity of the film would completely cover it.

On the other hand, the breathability of these films is based on a capillary porosity obtained by detaching the calcium carbonate particles from the polymeric matrix during the film post-stretching process; in order for this porous structure to preserve a capillary barrier capable of preventing leaks, the maximum pore size cannot exceed the capillary rise limit necessary to prevent the passage of fluid through the pore under the normal operating conditions for manufacturing the final product. This requires a pore size distribution that is as narrow as possible, since even a minimal portion of large-sized pores causes losses of fluids, thereby invalidating the main function of the final product. This phenomenon is exacerbated in warm climates, due to the effect of temperature on the surface tension of liquids, which leads to having to actually use films with surface weights greater than 30 g/m² for products used in these climates.

These and other disadvantages are resolved using the method and the product of the present invention.

### Description of the invention.

In the present description, the term "laminar product" refers to a product formed by a polymeric coating deposited over the surface of a substrate, wherein the "polymeric coating" consists of an adhesive polymeric layer.

Therefore, in the present description, the adhesive polymeric layer defines the polymeric coating of the invention and said terms will be used interchangeably to refer to the coating that is deposited over the non-woven fabric substrate.

In the present description, the term "breathability" refers to the water vapour permeability of the polymeric coating or the adhesive polymeric layer.

The method of the present invention makes it possible to solve the three main problems exixting in the production of covers of nappies and sanitary towels based on polymeric coatings deposited over a substrate using conventional technologies; namely: reducing the coating thickness, obtaining values for the breathability and the barrier against the loss of fluids that are independent from the pore size and the ambient temperature, and, finally, obtaining coatings that may be printed on the internal face of the product.

In essence, the method for obtaining a laminar product of the present invention comprises the following steps:
- Obtaining adhesive polymeric mixtures, where a base polymer is combined with a tackifying agent, in a mass proportion of the base polymer ranging between 60% and 95%;
- Feeding the adhesive polymeric mixtures in a melted state to a curtain-type applicator;
- Coating the substrate with the adhesive polymeric mixture, wherein the melted polymeric mixture is applied over the substrate in the form of a solid sheet, such that a laminar product is formed which is composed by a polymeric coating deposited over the non-woven fabric substrate, and wherein the polymeric coating consists of an adhesive polymeric layer with a thickness ranging between 1 and 10 micra;
- Winding of the laminar product obtained.

The laminar product obtained according to the method of the present invention comprises a polymeric coating that consists of an adhesive polymeric layer bonded, on one of its faces, to a non-woven fabric substrate, wherein said adhesive polymeric layer that forms the polymeric coating has a thickness value ranging between 1 and 10 micra, and a permeability value greater than 2000 g/(m²day), the laminar product having a total weight ranging between 10 and 20 g/m², and a thickness ranging between 10 and 15 micra.

In order to achieve the reduction in thickness of the laminar product of the present invention, the coating application technology known as "curtain coating" is used in the method for obtaining it.

The curtain coating technology is particularly suitable for applying reduced adhesive film thicknesses with a high precision. Said technology consists of a flat surface of a laminar substrate horizontally passing through a conveyor under a continuous flow of the polymeric coating material. It is worth mentioning the following examples of this type of applicators: the "cross coat" applicator, manufactured by Dynatec; the "curtain coat applicator", manufactured by Nordson; and the "slot curtain coater", manufactured by UNAV. This technology achieves continuous coating thicknesses that are substantially lower than the roughness height of the substrate, since it delays contact between the adhesive and the substrate in such a way that contact is made at high temperatures but in a solid state, after applying a stretching which reduces the thickness of the applied film.

However, this technology applied as such is not suitable for obtaining the desired polymeric coatings in this invention, for two reasons:
1^{st}- The polymeric coating must have adhesive capacity in the solid state; otherwise, it is not capable of adhering to the substrate. However, this adhesive capacity has the disadvantage that it is also present on the side opposite to the substrate, where it is not desirable, since, on the one hand, it slows down processing of the product on the production line, as the moving elements, such as the pinch or support rollers, become adhered, and, on the other hand, it makes unwinding of the product rolls impossible, as the coils become adhered to one another.
2^{nd}- On the other hand, the visco-elasticity qualities of the adhesive that is normally used in these applications, which, on the one hand, are partly necessary to achieve those reduced thicknesses, but ultimately cause the final product to have limited internal cohesion and, therefore, to create an insufficient hydraulic barrier for the desired application, i.e. for application as a coating in the manufacturing of nappies and sanitary towels.

In order to mitigate these disadvantages, the method of the invention anticipates using adhesive polymeric mixtures with the following characteristics:
- Strong adhesive capacity at high temperature, but much lower close to the ambient temperature.
- Adhesive capacity minimised by means of a plasma or corona treatment.
- Base polymer endowed with high elasticity in a melted state and, at the same time, an improved resistance to breaking.

These conditions are achieved by combining a base polymer with a tackifying agent according to the following characteristics:
The base polymer preferably consists of a polyolefin with a fluidity index ranging between 15 and 40 g/10 min, and preferably between 20 and 30 g/10 min, wherein said polyolefin is selected from the group formed by:
   - low-density polyethylene (LDPE), linear low-density polyethylene (LLDPE), polypropylene, ethylene-vinyl acetate (EVA) and the co-polymers thereof, ethylene- or propylene-based plastomers, such as ethylene/octene, ethylene/alphaolefin, ethylene/octane, ethylene/butane, and propylene/butylene co-polymers.

Likewise, favourable results have been obtained when using base polymers selected from the group formed by:
- thermoplastic polyurethanes, acrylic resins, styrene-butadiene resins or mixtures thereof, biodegradable polymers such as polylactic acid (PLA), polyhydroxyalkanoate (PHA), polycaprolactone (PCL), and mixtures thereof with polyolefins.

The base polymer will be dosed at a mass proportion ranging between 60% and 95%, and preferably between 80% and 90%.

On the other hand, the tackifying agent will be preferably selected on the basis of its compatibility with the selected base polymer and its sensitivity to the loss of adhesion caused by surface cross-linking, produced by means of corona, plasma or radiation treatments, as well as having suitable habitual technical parameters for the process and service conditions.

Thus, the tackifying agent is preferably selected from the group formed by:
- Hydrogenated aliphatic polyolefins, amorphous polyolefins, aromatic polyolefins, and tackifiers of the rosin and terpene types.

Using the method of the present invention, polymeric coatings with thicknesses ranging between 1 and 10 micra are obtained, which are significantly lower than the most reduced thicknesses found in the market. However, despite achieving these thickness values, the adhesive polymeric layer that forms the polymeric coating also maintains the necessary hydraulic barrier for the product.

It has been determined that, with the method described above, using a polymer that contains the necessary tackifying agent to achieve adhesion between the polymeric coating and the substrate causes the product to also have, at ambient temperature, has a residual adhesive capacity on the side of the polymeric coating opposite to that of the substrate. This residual adhesive capacity is very harmful, both during the subsequent processing of the laminar product bobbins, since adjacent layers of the bobbins adhere to one another, and in the final product processing machinery, since it causes adhesion to the machinery elements in contact with the product, which hinders or slows down the rate of use of this production equipment.

Therefore, another objective of the present invention is to substantially reduce this residual adhesive capacity of the product on the face of the polymeric coating that is not adhered to the substrate.

Said residual adhesion is reduced in four different ways, or by combining them in any manner:
- Using tackifying agents that are particularly sensitive to cross-linking of their molecule by means of radiation, plasma treatment or corona treatment, such as, for example, aliphatic polyolefins, and subjecting the side of the polymeric coating opposite to that of the substrate to a corona treatment of at least 30 dynes/cm, and more preferably of at least 40 dynes/cm; this treatment cross-links the molecule of the tackifying agent, causing it to lose a large part of its adhesive capacity.
- Selecting a tackifying agent with a significant adhesion/temperature gradient, such that it presents significant adhesion at the application temperature, which will range between 80ºC and 100ºC, and that this adhesive capacity is substantially reduced at ambient temperature, down to 60ºC. In order to achieve this effect, tackifying agents will be selected which have a softening temperature ranging between 80ºC and 100ºC.
- Performing a printing/decoration that covers most of the total surface area of the side of the polymeric coating that is not adhered to the substrate on the production line, prior to winding the product, by means of any known continuous strip printing method, such as flexography or photogravure.
- Adding a second coating layer that becomes adhered to the first using its residual adhesive capacity, but which, in itself, lacks adhesive capacity. This second layer is preferably added at the same time as the adhesive polymeric layer that defines the polymeric coating, using a dual-layer curtain applicator, but it may also be added using a second applicator.

On the other hand, another important element of the invention are the breathability values of the polymeric coating, which are of special interest for the desired application of these coatings.

As mentioned above, using the method of the invention, breathable polymeric coatings may be obtained based solely on the natural water vapour permeability of the base polymer, without the need to create pores in the polymer by means of the habitual methods based on the post-stretching of polymeric films loaded with calcium carbonate particles.

Taking into consideration that the target breathability in the market ranges between 2000 and 3000 g/(m²day) (38ºC, 90% RH, ASTM 398), and that, using the method of the present invention, coatings with thicknesses of the adhesive polymeric coating layer ranging between 1 and 3 micra are usually obtained, said permeability values are achieved by using base polymers with a natural water vapour permeability (based on absorption-diffusion-desorption mechanisms) ranging between 3 and 9 g/(m²day). In this regard, a significant number of base polymers meet this permeability; for example, without being limited thereto, the following polymers, which have permeability values within the specified range, may be used as base polymers:
- Polyamides: between 5 and 10 g/(m²day).
- Polystyrene: between 1 and 4 g/(m²day).
- EVA-co-polymers: between 3.5 and 5 g/(m²day).
- ABS (Acrylonitrile/butadiene/styrene): between 3 and 6 g/(m²day).
- Polyethylene terephthalate (PET): between 1 and 4 g/(m²day).
- Thermoplastic polyurethanes (PU):
- Thermoplastic co-polyamide elastomers:
- Polylactic acid (PLA): between 2 and 4 g/(m²day).

Moreover, when base polymers with an intrinsic permeability of less than 3 g/(m²day) are used, such as, for example, polyolefins, which are particularly suitable for this application due to their low cost and their availability in the market, the water vapour permeability of the base polymer substantially increases by adding, to the composition thereof, hydrophilic agents made up of polymers or oligomers with highly hydrophilic polar groups in their molecules, combined with adhesive/dispersing agents made up of co-polymers of the base polymer, grafted with polar groups fixed onto the polymeric matrix. These adhesive/dispersing agents are essential to ensure a stable, homogeneous dispersion of the hydrophilising agent in the base polymer. In the event that only moderate increases in water vapour permeability are required, the presence of the adhesive/dispersing agent, in a proportion ranging between 2% and 20% of the base polymer, without the hydrophilising agent, is sufficient.

To this end, polyethylene glycol (PEG), which has a molecular weight ranging between 500 and 10000, and preferably between 5000 and 10000, is particularly suitable, due its effectiveness, its availability in the market, its innocuousness and its low cost, since the molecule must have sufficient volume to prevent its migration to the surface of the polymer, but not so much so as to prevent the stable formation of a complete, homogeneously dispersed network of hydrophilic points distributed inside the polymer, this hydrophilising agent being dosed at a proportion ranging between 3% and 20% of the base polymer. The PEG must be combined with an adhesive/dispersing agent made up of an adhesive co-polymer of the base polymer containing grafted polar groups, such as, for example, without being limited thereto, maleic anhydride, acrylic acid, acrylates or acetates, glycidyl acetate derivatives, as well as mixtures thereof, which will act as dispersants for the hydrophilic agent and fix the position of the PEG molecules in order to prevent them from migrating to the surface of the polymer and coalescing. This adhesive/dispersing agent must be dosed at a proportion ranging between 1% and 5% of the base polymer.

On the other hand, with regards to the printing of the polymeric coating, it is worth mentioning that, in addition to conducting said printing as a method for eliminating the residual adhesiveness of the coating, as mentioned above, this type of products are usually printed in the market. This type of printed products present in the market are composed of a printed PE sheet on the side that is adhered to the non-woven fabric substrate, such that the printing may be seen through the non-woven fabric layer. In the case of products coated by means of direct extrusion, since it is not possible to print the side of the coating that is adhered to the substrate, the non-woven fabric must be printed, which causes a loss of quality in the printing, due to the substrate roughness.

In the present invention, the actual reduced thickness of the coating provides sufficient transparency to allow for printing on the side of the polymeric coating that is not adhered to the substrate, without any decrease in the quality of the printing.

Other details and characteristics will be presented throughout the following description, wherein, for illustrative, non-limiting purposes, an embodiment of the invention is shown, by referring to the drawings attached to this specification.

### Description of the figures.

Below we include a list of the different parts of the invention, which, with the aid of the corresponding numbers, are found in the figures: (1) unwinder, (2) continuous mixer, (3) curtain-type applicator, (4) printing equipment, (5) winder, (10) laminar product, (11) polymeric coating, (11 a) side of the polymeric coating, (12) substrate, (13) second coating layer, (21) heated pipe.
Figure no. 1 is a schematic representation of the application line of the method of the invention, which comprises a non-woven fabric unwinder (1), a curtain applicator (2) and a finished product winder (5).
Figure no. 2 is a schematic representation of the step for coating the substrate with the polymeric mixture.
Figure no. 3 corresponds to a schematic representation of the application of another polymeric layer without adhesive capacity.
Figure no. 4a is a schematic representation of the thickness profile of the polymeric coating placed over the substrate with the curtain-type coating applicator used in the invention.
Figure no. 4b is a schematic representation of the thickness profile of the polymeric coating placed over the substrate using a conventional method.

### Preferred embodiment of the invention.

The method of the proposed invention involves producing a laminar product (10) formed by a polymeric coating (11) placed over a non-woven fabric substrate (12), said laminar product (10) being particularly suitable for manufacturing external covers of sanitary towels and nappies.

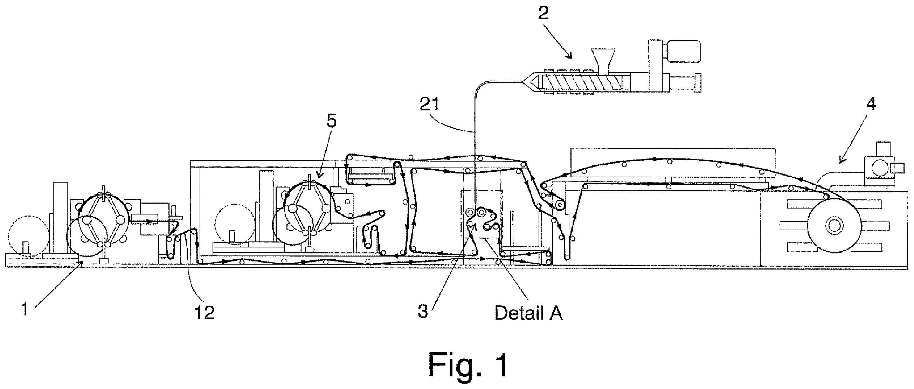

As shown in Figure No. 1, the method of the invention is performed on an application line (production line) that comprises a non-woven fabric unwinder (1), a curtain-type applicator (3) for the adhesive polymeric mixture, and a winder (5) for the finished product, i.e. the final laminar product.

In essence, the method of the invention comprises the following steps:
a)- Obtaining adhesive polymeric mixtures, wherein a base polymer is combined with a tackifying agent, in a mass proportion ranging between 60% and 95% of the base polymer. In this step, the polymeric mixture is manufactured in continuous mixing equipment (co-rotating twin-screw extruders; single-screw extruders-mixers; Buss-type continuous mixers); or batch equipment (Brabender-type batch mixers). The polymeric mixture may be manufactured in line with the production, as shown in Figure no. 1, wherein a continuous mixer (2) is illustrated, or separated in time, in a previous step, by storing the mixture in the form of pellets that will be pumped into the main line once they are melted.
b)- Feeding the melted mixtures to a curtain-type applicator (3). In this step, the curtain applicator is fed by pumping the melted mixture obtained in step "a" through a heated pipe (21).
c)- Coating the substrate (12) with the polymeric mixture, wherein the melted polymeric mixture is applied over the non-woven fabric substrate (12) by means of a curtain-type coating applicator (3), such that a laminar product (10) is created which is formed by a polymeric coating (11), consisting of an adhesive polymeric layer placed over the non-woven fabric substrate (12), wherein the adhesive polymeric layer has a thickness ranging between 1 and 10 micra, and is homogeneously distributed, with a constant thickness, over the surface of the substrate. This step is performed by means of the applicator (3), using equipment such as, for example, equipment from the German manufacturer Dynatec.
d)- Winding of the laminar product (10). In this step, the laminar product (10) obtained is wound using the winder (5).

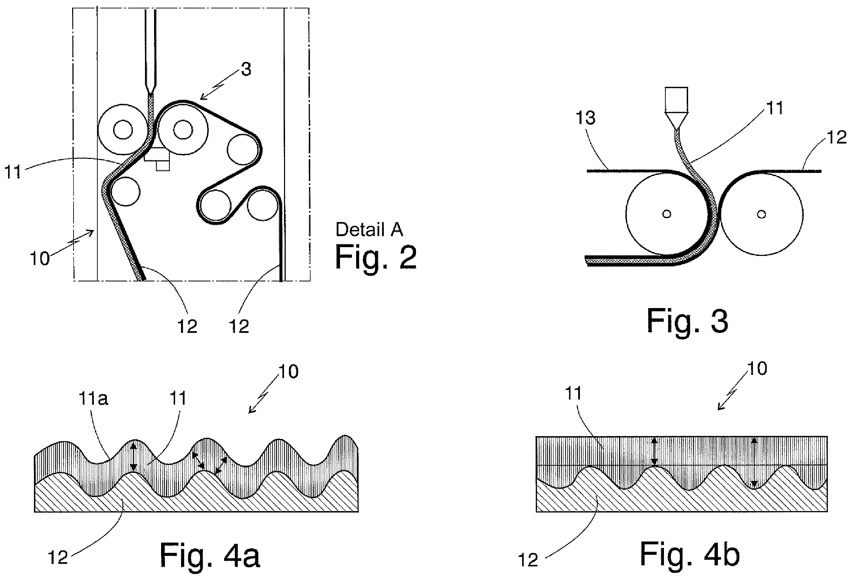

As illustrated in Figure No. 2, in step c), the melted polymeric mixture is applied, in the form of a sheet, over the non-woven fabric substrate (12) on the application line shown in Figure No. 1.

Application of the melted polymeric mixture in the form of a sheet guarantees that the adhesive polymeric layer that forms the polymeric coating has a uniform thickness throughout the entire substrate (12), as illustrated in Figure No. 4a, which shows the thickness profile of the adhesive polymeric layer when it is applied over the substrate using the curtain technique according to the present invention, unlike what is shown in Figure 4b, wherein the adhesive polymeric layer that forms the polymeric coating is applied using conventional techniques in the prior art.

When comparing the two figures, Figures 4a and 4b, it may be observed that, using the method of the present invention, a smaller quantity of adhesive polymeric material is used to form the adhesive polymeric layer that forms the coating (11), unlike what happens when traditional application methods are used, all of which entails considerable savings in polymeric material and the production of a laminar product (10) with a smaller thickness and a lower weight.

In essence, the technology used in the present invention makes it possible to obtain a polymeric coating (11) with a thickness ranging between 1 and 10 micra and, therefore, makes it possible to obtain a laminar product (10) with a weight ranging between 10 and 30 g/cm², which is substantially lower than the weight of similar products found in the prior art.

Moreover, the adhesive polymeric layer that forms the polymeric coating (11), uniformly distributed over the substrate, is impermeable to liquid water, but permeable to water vapour, with permeability values greater than 2000 g/(m²day).

In addition, in order to substantially reduce the residual adhesive capacity of the side (11 a) of the polymeric coating (11), i.e. the side of the adhesive polymeric layer that is not adhered to the substrate (12), one or a combination of the following steps are performed:
- Printing on the side of the polymeric coating (11 a) that is not adhered to the substrate, wherein said printing (4) may be performed on the application line prior to the winding step, as illustrated in Figure no. 1, or in a subsequent treatment of the coated product bobbins, using a method selected from photogravure, flexography or transfer printing.
- Applying a second coating (13) without adhesive capacity over the side (11 a) of the polymeric coating (11) that is not bonded to the substrate. This step may be performed at the same time as the coating of the substrate with the polymeric mixture, using a dual-layer curtain applicator for this purpose, as illustrated in Figure no. 3, or subsequently, by means of a second applicator.

- Performing a cross-linking treatment of the tackifying agent, selected from: corona treatment, ranging between 30 dynes/cm and 50 dynes/cm, radiation treatment, or plasma treatment.

On the other hand, in order to increase the breathability of the polymeric coating (11), thereby increasing its water vapour permeability to values greater than 2000 g/(m²day), during the step for obtaining the polymeric mixtures, hydrophilic agents are added in a proportion ranging between 3% and 20% of the base polymer, consisting of polymers or oligomers with highly hydrophilic polar groups in their molecules, polyethylene glycol, with a weight ranging between 500 and 5000, being particularly suitable for this purpose, and combined with adhesive/dispersing agents consisting of co-polymers of the base polymer, grafted with polar groups selected from the group formed by: maleic anhydride, acrylic acid, acrylates or acetates, glycidyl acetate derivatives, as well as mixtures thereof, dosed at a proportion ranging between 1% and 5% of the base polymer.

Other hydrophilic agents may also be used, such as, for example, polyvinyl alcohol, polyvinyl acetate, polyethylene oxides, polypropylene glycols and thermoplastic polyurethanes.

In the event that only moderate increases in permeability are required, it is sufficient to add the adhesive/dispersing agent in a proportion ranging between 2% and 20%, without the need to add the hydrophilising agent.

Following the method described in the present invention, laminar products with the following characteristics were obtained:

### Example 1.

Cross-coat contactless coating equipment manufactured by Dynatec.
Substrate: Spun-bond non-woven fabric of 13 g/m² Polypropylene (PP), manufactured by PGI.
Polymeric coating: Ethylene-vinyl-acetate (EVA)-based adhesive, ref. Pergimelt 21340, manufactured by Wetzel, 3 g/m².
Flexographic printing: 1 g/m².
Total weight of the product: 17 g/m².
Corona treatment: 46 dynes/cm.

### Example 2.

Substrate: Spun-bond non-woven fabric of 13 g/m² "PP", manufactured by PGI.
Polymeric coating: "EVA"-based adhesive, ref. Pergimelt 21340, manufactured by Wetzel: 2 g/m².
Flexographic printing: 1 g/m².
Total weight of the product: 16 g/m².
Corona treatment: 46 dynes/cm.

### Example 3.

Substrate: Spun-bond non-woven fabric of 12 g/m² "PP", manufactured by Texbond.
Polymeric coating: "PP"-based adhesive, ref. Technomelt 2146, manufactured by Henkel: 2 g/m².
Flexographic printing: 1 g/m².
Total weight of the product: 15 g/m².
Corona treatment: 46 dynes/cm.

### Example 4:

Substrate: Spun-bond non-woven fabric of 10 g/m² "PP", manufactured by Texbond.
Polymeric coating, 2 g/m²: Thermoplastic elastomer Engage 8402, manufactured by Dow: 80%; tackifying agent Eastotac H130W, manufactured by Eastman: 20%.
Flexographic printing: 1 g/m².
Total weight of the product: 13 g/m².
Corona treatment: 46 dynes/cm.

### Example 5:

Substrate: Spun-bond non-woven fabric of 10 g/m² "PP", manufactured by Texbond.
Polymeric coating, 3 g/m²: Thermoplastic elastomer Engage 8402, manufactured by Dow: 77%; tackifying agent Regalite R1125, manufactured by Eastman: 20%; low-density-polyethylene (LDPE)-acrylic co-polymer Primacor 3460, manufactured by Dow: 3%.
Flexographic printing: 1 g/m².
Total weight of the product: 14 g/m².
Corona treatment: 46 dynes/cm.

### Example 6:

Substrate: Spun-bond non-woven fabric of 10 g/m² "PP", manufactured by Texbond.
Polymeric coating, 3 g/m²: Thermoplastic elastomer Engage 8402, manufactured by Dow: 72%; tackifying agent Regalite R1125, manufactured by Eastman: 20%; "LDPE"-acrylic co-polymer Primacor 3460, manufactured by Dow: 3%; polyethylene glycol (PEG) 3350 Carbowax, manufactured by Dow: 5%.
Flexographic printing: 1 g/m².
Total weight of the product: 14 g/m².
Corona treatment: 46 dynes/cm.

### Example 7:

Substrate: Spun-bond non-woven fabric of 10 g/m² "PP", manufactured by Texbond.
Polymeric coating: Thermoplastic elastomer Engage 8402, manufactured by Dow: 77%; tackifying agent Eastotac H130W, manufactured by Eastman: 20%; acrylic co-polymer Primacor 3460, manufactured by Dow: 3%: 1 g/m².
Flexographic printing: 1g/m².
Total weight of the product: 12 g/m².
Corona treatment: 46 dynes/cm.

Table no. 1 shows a summary of the values for the total weight of the laminar product obtained, the weight of the polymeric coating, as well as the total barrier and the breathability (water vapour permeability), obtained for each of the products in the examples.

**Table no. 1:**

| | **Ex.1** | **Ex.2** | **Ex.3** | **Ex.4** | **Ex.5** | **Ex.6** | **Ex.7** |
|---|---|---|---|---|---|---|---|
| **Total weight of the product g/m²** | 17 | 16 | 15 | 13 | 14 | 14 | 12 |
| **Weight of the polymeric coating g/m²** | 3 | 2 | 2 | 2 | 3 | 3 | 1 |
| **Total barrier of the product mm of H₂O** | 584 | 382 | 1151 | 1504 | 2356 | 2242 | 504 |
| **Breathability of the product g/(m²day)** | 2324 | 3502 | 10 | 21 | 1937 | 4841 | 5802 |

As illustrated in said Table no. 1, all the products obtained had a total weight ranging between 10 and 30 g/m², with permeability values greater than 2000 g/(m²day), except in the case of Examples 3 and 4, the low permeability values whereof may be explained because their base polymers, polypropylene and polyethylene, respectively, have very low natural permeability values, 0.2 and 0.45 g/(m²day), respectively. In example 5, we may observe the effect of adding the adhesive/dispersing agent, in this case Primacor 3460, manufactured by Dow, an ethylene-acrylic acid co-polymer, which increases the permeability from 21 g/(m²day) to about 2000 g/(m²day), and, in Example 6, we may observe that the permeability increases to about 5000 g/(m²day) by also adding the hydrophilising agent polyethylene glycol Carbowax 3350, manufactured by Dow.

Moreover, as may be observed in Table no. 1, the weights of the polymeric coatings obtained range between 1 and 3 g/m²; this is achieved thanks to the method of application of the polymeric coating over the substrate, by means of the curtain technology, which achieves uniform distribution of an adhesive polymeric layer over the substrate, with a thickness ranging between 1 and 10 micra. This entails a considerable reduction in the quantities of polymeric material used to produce the coatings of nappies and sanitary towels, and, consequently, considerable savings in the production costs of these products.

Having sufficiently described the present invention, in line with the embodiments explained, it is evident that those modifications deemed convenient may be made to details thereof, provided that they do not alter the essence of the invention, which is summarised in the following claims.

## Claims

1. Method for producing a laminar product particularly suitable for manufacturing external covers of sanitary towels and nappies, of the type formed by a polymeric coating deposited over a non-woven fabric substrate, **characterised in that** it comprises the following steps:
- Obtaining adhesive polymeric mixtures, wherein a base polymer is combined with a tackifying agent in a mass proportion ranging between 60% and 95% of the base polymer;
- Feeding the mixtures, in a melted state, to a curtain-type coating applicator;
- Coating the substrate (12) with the polymeric mixture, wherein the melted polymeric mixture is applied over the substrate (12) by means of the curtain-type coating applicator, such that a laminar product (10) is formed that is composed by a polymeric coating (11) consisting of an adhesive polymeric layer, which is deposited over the non-woven fabric substrate (12), wherein the adhesive polymeric layer that forms the polymeric coating (11) has a thickness ranging between 1 and 10 micra;
- Winding the laminar product obtained, the laminar product (10) having a total weight ranging between 10 and 30 g/m², and permeability values greater than 3000 g/(m²day).

2. Method according to claim 1, wherein, in the step for obtaining the adhesive polymeric mixtures, the base polymer that is combined is a polyolefin selected from the group formed by:
- low-density polyethylene (LDPE), linear low-density polyethylene (LLDPE), polypropylene, ethylene-vinyl acetate (EVA) and co-polymers thereof, ethylene- or propylene-based plastomers such as ethylene/octene, ethylene/alphaolefin, ethylene/octane, ethylene/butane, and propylene butylene co-polymers.

3. Method according to claim 1, wherein, in the step for obtaining the adhesive polymeric mixtures, the base polymer that is combined is selected from the group formed by:
- thermoplastic polyurethanes, acrylic resins, styrene-butadiene resins or mixtures thereof, biodegradable polymers, such as polylactic acid (PLA), polyhydroxyalkanoate (PHA), polycaprolactone (PCL), and mixtures thereof with synthetic polymers, polyamides, polystyrene, ethylene-vinyl acetate (EVA) co-polymers, acrylonitrile/butadiene/styrene (ABS) co-polymers, polyethylene terephthalate (PET), polyurethane (PU), and polylactic acid (PLA).

4. Method according to any of claims 1 to 3, wherein, in the step for obtaining the adhesive polymeric mixtures, the tackifying agent is selected from the group formed by:
- Hydrogenated aliphatic polyolefins, amorphous polyolefins, aromatic polyolefins, and tackifying agents of the rosin and terpene types.

5. Method according to claim 1, wherein, in the step for obtaining the adhesive polymeric mixtures, when the base polymer has natural water vapour permeability values lower than 3 g/(m²day), in order to increase the permeability, to said base polymer it is added a hydrophilising agent selected from the group formed by highly hydrophilic polymers, such as: polyvinyl alcohol, polyvinyl acetate, polyethylene oxides, polypropylene glycols and thermoplastic polyurethanes, which have highly hydrophilic polar groups in their molecules, and where the hydrophilising agent is dosed at a proportion ranging between 3% and 20% of the base polymer; and wherein an adhesive/dispersant agent is added, selected from the co-polymers of the base polymer, grafted with polar groups selected from the group formed by: maleic anhydride, acrylic acid, acrylates or acetates, glycidyl acetate derivatives, as well as mixtures thereof, where the adhesive/dispersant agent is dosed at a proportion ranging between 1% and 5% of the base polymer.

6. Method according to claim 5, wherein the highly hydrophilic polar group is polyethylene glycol, with a weight ranging between 500 and 1000.

7. Method according to claim 1, wherein, in the step for obtaining the adhesive polymeric mixtures, when the base polymer has natural water vapour permeability values lower than 3 g/(m²day), in order to increase the permeability, only an adhesive/dispersing agent is added, dosed at a proportion ranging between 2% and 20% of the base polymer, without the presence of a hydrophilising agent.

8. Method according to claim 1, comprising an additional step for printing (4) on the side (11 a) of the polymeric coating (11) that is not adhered to the substrate, where said printing (4) is performed on the production line prior to the winding, by means of a method selected from photogravure, flexography or transfer printing.

9. Method according to claim 1, comprising an additional step for applying a second coating (13) without adhesive capacity over the side (11 a) of the polymeric coating (11) that is not bonded to the substrate (12).

10. Method according to claim 1, wherein, prior to the winding, the laminar product is subjected to a cross-linking treatment selected from the group formed by: corona treatment, ranging between 30 dynes/cm and 45 dynes/cm, radiation treatment, or plasma treatment.

11. Method according to claim 1, wherein the obtention of the polymeric mixture is performed by extrusion, and may be performed on the application line or in a separate step.

12. Method according to claim 8, wherein the printing is performed on the application line or outside said line, as a treatment of the product bobbins.

13. Method according to claim 1, wherein the polymeric mixtures are made in continuous mixing equipment, such as: co-rotating twin-screw extruders, single-screw extruders with mixing elements, Buss-type continuous mixers; or in batch mixing equipment.

14. Method according to claim 1, wherein the application line comprises a non-woven fabric unwinder (1), a curtain-type coating applicator (2), and a winder (25) for the laminar product obtained.

15. Laminar product obtained according to claims 1 to 14, suitable for manufacturing external covers of sanitary towels and nappies, comprising a polymeric coating (11) bonded on one of the faces thereof, to a non-woven fabric substrate (12), wherein said coating consists of an adhesive polymeric layer that has a thickness value ranging between 1 and 10 micra, the laminar product (10) having a total weight ranging between 10 and 30 g/m², and permeability values greater than 2000 g/(m²day).

16. Laminar product according to claim 15, wherein the adhesive polymeric layer comprises a base polymer plus a tackifying agent, and wherein the base polymer is a polyolefin dosed at a mass proportion ranging between 60% and 95%.

17. Laminar product according to claim 16, wherein the polyolefin is selected from the group formed by:
- low-density polyethylene (LDPE), linear low-density polyethylene (LLDPE), polypropylene, ethylene-vinyl acetate (EVA) and co-polymers thereof, ethylene- or propylene-based plastomers, such as ethylene/octene, ethylene/alphaolefin, ethylene/octane, ethylene/butane, and propylene/butylene co-polymers.

18. Laminar product according to claim 15, wherein the base polymer is selected from the group formed by:
- thermoplastic polyurethanes, acrylic resins, styrene-butadiene resins, or mixtures thereof, biodegradable polymers, such as polylactic acid (PLA), polyhydroxyalkanoate (PHA), polycaprolactone (PCL), and mixtures thereof with synthetic polymers, polyamides, polystyrene, ethylene-vinyl acetate (EVA) co-polymers, acrylonitrile/butadiene/styrene (ABS) co-polymers, polyethylene terephthalate (PET), polyurethane (PU), and polylactic acid (PLA).

19. Laminar product according to claims 15 to 18, wherein the tackifying agent is selected from the group formed by:
- Hydrogenated aliphatic polyolefins, amorphous polyolefins, aromatic polyolefins, and tackifying agents of the rosin and terpene types.

20. Laminar product according to claim 19, wherein the tackifying agent is selected with a softening temperature ranging between 80ºC and 100ºC.

21. Laminar product according to claim 16, wherein, when the base polymer has natural water vapour permeability values lower than 3 g/(m²day), in addition to the tackifying agent, a hydrophilising agent is added to said base polymer which is selected from the group formed by highly hydrophilic polymers, such as: polyvinyl alcohol, polyvinyl acetate, polyethylene oxides, polypropylene glycols and thermoplastic polyurethanes, which have highly hydrophilic polar groups in their molecules, where the hydrophilising agent is dosed at a proportion ranging between 3% and 20% of the base polymer; and wherein an adhesive/ dispersant agent is added, which is selected from co-polymers of the base polymer, grafted with polar groups selected from the group formed by: maleic anhydride, acrylic acid, acrylates or acetates, glycidyl acetate derivatives, as well as mixtures thereof, where the adhesive/dispersant agent is dosed at a proportion ranging between 1% and 5% of the base polymer.

22. Laminar product according to claim 21, wherein the highly hydrophilic polar group is polyethylene glycol, with a weight ranging between 500 and 1000.

23. Laminar product according to claim 16, wherein, when the base polymer has natural water vapour permeability values lower than 3 g/(m²day), in order to increase the permeability, only an adhesive/dispersing agent is added, dosed at a proportion ranging between 2% and 20% of the base polymer, without the presence of a hydrophilising agent.

24. Laminar product according to claim 15, wherein the side (11 a) of the polymeric coating (11) that is not adhered to the substrate (12) has a printing or decoration performed by means of a continuous strip printing method, such that the residual adhesion of said side disappears.

25. Laminar product according to claim 15, comprising a second coating layer (13) without adhesive capacity, adhered to the side (11 a) of the polymeric coating (11) that is not bonded to the substrate.
